# EUROPEAN PATENT APPLICATION

(11) **EP 1 552 832 A1**
(43) Date of publication of application: **13.07.2005**
(21) Application number: 03741327.5
(22) Date of filing: 10.07.2003
(51) Int. Cl.: A61K 31/4439, A61K 31/155, A61K 9/32, A61K 45/00, A61K 47/08, A61K 47/10, A61P 3/10, A61P 43/00

(54) **PROCESS FOR PRODUCING COATED PREPARATION**

(30) Priority: 11.07.2002 JP 2002203119
(71) Applicant: Takeda Pharmaceutical Company Limited, Osaka 541-0045 (JP)
(72) Inventor: KOIKE, Masahiko, Toyonaka-shi, Osaka 560-0021 (JP); KOYAMA, Hiroyoshi, Shimamoto-cho, Mishima-gun, Osaka 618-00 (JP); HAMAGUCHI, Naoru, Ibaraki-shi, Osaka 567-0832 (JP)
(74) Representative: Rickard, Timothy Mark Adrian
(86) International application number: PCT/JP2003/008790
(87) International publication number: WO 2004/006921

(57) **Abstract**

The present invention provides a production method of a preparation coated with pioglitazone hydrochloride, which is useful as a therapeutic agent for diabetes and the like and superior in the characteristics of the preparation such as dissolution property of pioglitazone hydrochloride and the like.

## Description

### TECHNICAL FIELD OF THE INVENTION

The present invention relates to a production method of a preparation coated with pioglitazone hydrochloride, which is useful as a therapeutic agent for diabetes and the like.

### BACKGROUND ART

There are the following reports on pharmaceutical compositions containing an insulin sensitizer such as a thiazolidinedione compound and the like and a biguanide.
1) EP-A-749751 reports a pharmaceutical agent containing an insulin sensitizer in combination with at least one member from an α-glucosidase inhibitor, an aldose reductase inhibitor, a biguanide, a statin compound, a squalene synthesis inhibitor, a fibrate compound, an LDL catabolism enhancer and an angiotensin converting enzyme inhibitor.
2) WO98/57634 reports a pharmaceutical composition containing an insulin sensitizer, a biguanide antihyperglycaemic agent and a pharmaceutically acceptable carrier.
3) WO01/35940 reports a pharmaceutical composition containing thiazolidinedione, metformin hydrochloride and a pharmaceutically acceptable carrier, wherein thiazolidinedione is formulated on the surface of metformin hydrochloride.
4) WO01/35941 reports a pharmaceutical composition containing thiazolidinedione, metformin hydrochloride and a pharmaceutically acceptable carrier, wherein thiazolidinedione and metformin hydrochloride are respectively dispersed in pharmaceutically acceptable carriers of their own.
5) WO01/82875 reports a core formulation comprising (a) a first layer containing pioglitazone hydrochloride or a pharmaceutically acceptable salt thereof as an active ingredient, and (b) a core containing a biguanide as an active ingredient, wherein at least a part of the core is enclosed by said first layer.
6) USP6403121 reports a core formulation comprising a first layer containing pioglitazone hydrochloride, which covers at least a part of a core containing a biguanide, wherein one or both of the core and the first layer is/are dispersed in a modulating release agent such as polysaccharides and the like.

### DISCLOSURE OF THE INVENTION

There is a demand for the development of a production method of a preparation coated with pioglitazone hydrochloride, which is useful as a therapeutic agent for diabetes and the like and superior in the characteristics of the preparation such as dissolution property of pioglitazone hydrochloride and the like.

The present inventors have found, in producing a preparation coated with pioglitazone hydrochloride, that a coated preparation that shows superior dissolution property of pioglitazone hydrochloride can be obtained by coating with a dispersion of pioglitazone hydrochloride in an organic solvent (an organic solvent dispersion of pioglitazone hydrochloride), which contains a coating base soluble in organic solvents. The present inventors have further studied based on this finding and completed the present invention.

Accordingly, the present invention relates to
1) a production method of a coated preparation, which comprises coating with a dispersion of pioglitazone hydrochloride in an organic solvent, which contains a coating base soluble in organic solvents;
2) a coated preparation obtained according to the production method of the aforementioned 1);
3) the production method of the aforementioned 1), which comprises coating a core containing an active ingredient with a dispersion of pioglitazone hydrochloride in an organic solvent, which contains a coating base soluble in organic solvents;
4) the production method of the aforementioned 3), wherein the active ingredient is a therapeutic agent for diabetes;
5) the production method of the aforementioned 4), wherein the therapeutic agent for diabetes is a biguanide;
6) the production method of the aforementioned 5), wherein the biguanide is metformin hydrochloride;
7) the production method of the aforementioned 1), wherein the organic solvent is an alcohol or a ketone;
8) the production method of the aforementioned 1), wherein the organic solvent is ethanol;
9) the production method of the aforementioned 1), wherein the coating base soluble in organic solvents is polyvinylpyrrolidone;
10) a method for improving dissolution of pioglitazone hydrochloride from a preparation coated with pioglitazone hydrochloride, which comprises, when producing said preparation, coating with a dispersion of pioglitazone hydrochloride in an organic solvent, which contains a coating base soluble in organic solvents;
11) a coated preparation obtained according to the production method of the aforementioned 1), which shows elution of not less than 50% of pioglitazone hydrochloride in 15 minutes in a dissolution test by a rotating basket method using a hydrochloric acid-potassium chloride buffer (pH 2.0) as a test solution at 37°C, 100 rpm;
12) a coated preparation obtained according to the production method of the aforementioned 1), which shows elution of not less than 50% of pioglitazone hydrochloride in 15 minutes in a dissolution test by a puddle method using a hydrochloric acid-potassium chloride buffer (pH 2.0) as a test solution at 37°C, 50 rpm; and the like.

The average particle size of pioglitazone hydrochloride used in the present invention is preferably 0.5-500 µm, more preferably 1-150 µm.

The organic solvent dispersion to be used in the present invention may be an organic solvent solution or an organic solvent suspension.

As the organic solvent, for example, alcohols such as methanol, ethanol, propyl alcohol, isopropyl alcohol, 2-methyl-1-propanol and the like; ketones such as acetone, methyl ethyl ketone, methyl isobutyl ketone and the like; esters such as methyl acetate, ethyl acetate, propyl acetate, isopropyl acetate, ethyl formate and the like; hydrocarbons such as heptane and the like; halogenated hydrocarbons such as dichloromethane and the like; and the like can be mentioned. These organic solvents may be used in a mixture of two or more kinds thereof in an appropriate ratio. The organic solvent may contain water. When the organic solvent contains water, the proportion of water to the organic solvent is, for example, not more than 50% (W/W), preferably not more than 30% (w/w), more preferably not more than 20% (W/W).

The organic solvent is preferably an alcohol or a ketone, and is more preferably methanol, ethanol, propyl alcohol, isopropyl alcohol, acetone and the like. Of these, ethanol is preferable.

The concentration of pioglitazone hydrochloride in the organic solvent dispersion is, for example, 1-25% (W/W), preferably 1-15% (W/W). Concentrations in these ranges are preferable in view of workability of coating, content uniformity of pioglitazone hydrochloride in the obtained coated preparation, and the like.

The "dispersion of pioglitazone hydrochloride in an organic solvent" (hereinafter sometimes to be abbreviated as a dispersion of the present invention) contains a coating base soluble in organic solvents.

As used herein, the coating base soluble in organic solvents means a coating base capable of dissolving in a mixture of the aforementioned organic solvent (the organic solvent may be a mixture of two or more kinds thereof and may contain water).

In the present specification, by being "soluble in organic solvents" is meant a property to dissolve in an organic solvent in a proportion of not less than 2% (W/V) at, for example, room temperature (preferably 20°C).

As the "coating base soluble in organic solvents", for example, cellulose polymers (e.g., hydroxypropyl cellulose, hydroxyethyl cellulose and the like); synthetic polymers (e.g., polyvinylacetal diethylaminoacetate [AEA (trademark), SANKYO CO., LTD.], aminoalkylmethacrylate copolymer E [Eudragit E (trademark), Rohm Pharma], polyvinylpyrrolidone and the like); and the like can be mentioned.

The above-mentioned coating base may be a mixture of two or more kinds thereof in an appropriate ratio.

The coating base soluble in organic solvents is preferably polyvinylpyrrolidone, hydroxypropyl cellulose and the like, more preferably polyvinylpyrrolidone.

The coating base soluble in organic solvents may be dissolved or suspended in the dispersion of the present invention. For efficient production of a coated preparation superior in content uniformity of pioglitazone hydrochloride and strength of the preparation, the coating base is preferably dissolved in the dispersion of the present invention.

The dispersion of the present invention may further contain a coating additive. As the coating additive, for example, shading agents and/or coloring agents such as titanium oxide, talc, ferric oxide and the like; plasticizers such as polyethylene glycol, triethyl citrate, castor oil, polysorbates and the like; organic acids such as citric acid, tartaric acid, malic acid, ascorbic acid and the like; lactose, D-mannitol, low-substituted hydroxypropyl cellulose, carmellose calcium, crospovidone and the like can be mentioned.

When the coating additive is not soluble in organic solvents, the average particle size thereof is preferably not more than 500 µm, more preferably not more than 150 µm, particularly preferably not more than 75 µm. When a coating additive having such average particle size is used, a coated preparation superior in content uniformity of pioglitazone hydrochloride and strength of the preparation can be obtained efficiently.

The concentration of the coating base soluble in organic solvents in the dispersion of the present invention is, for example, 1-25% (W/W), preferably 2-20% (W/W). Concentrations in these ranges are preferable in view of workability of coating, content uniformity of pioglitazone hydrochloride in the obtained coated preparation, and the like.

The concentration of the coating additive in the dispersion of the present invention is, for example, 0.2-25% (W/W), preferably 0.5-15% (W/W). Concentrations in these ranges are preferable in view of workability of coating, content uniformity of pioglitazone hydrochloride in the obtained coated preparation, and the like.

As the core to be coated with a dispersion of pioglitazone hydrochloride in an organic solvent, which contains a coating base soluble in organic solvents (hereinafter sometimes to be abbreviated as a core of the present invention), for example, solid preparations such as tablet, capsule, granule, powder, troche and the like can be mentioned. The solid preparation may be a controlled release preparation such as quick release preparation, release sustaining preparation (sustained release preparation) and the like. The solid preparation may contain a conventional additive in the field of pharmaceutical preparation and can be also produced according to a known method. As the additive, for example, excipient, disintegrant, binder, lubricant, coloring agent, pH regulator, surfactant, release-sustaining agent, stabilizer, sour agent, flavor, fluidizing agent and the like can be mentioned. These additives are used in an amount conventionally employed in the field of pharmaceutical preparation.

As the excipient, for example, starches such as corn starch, potato starch, wheat starch, rice starch, partly pregelatinized starch, pregelatinized starch, porous starch and the like; sugars and sugar alcohols such as lactose, fructose, glucose, D-mannitol, sorbitol and the like; anhydrous calcium phosphate, crystalline cellulose, precipitated calcium carbonate, calcium silicate and the like can be mentioned.

As the disintegrant, for example, carboxymethyl cellulose, calcium carboxymethyl cellulose, sodium carboxymethyl starch, croscarmellose sodium, crospovidone, low-substituted hydroxypropyl cellulose, hydroxypropyl starch and the like are used. The amount of the disintegrant to be used is preferably 0.5-25 parts by weight, more preferably 1-15 parts by weight, per 100 parts by weight of the solid preparation.

As the binder, for example, crystalline cellulose, hydroxypropyl cellulose, hydroxypropylmethyl cellulose, polyvinylpyrrolidone, gum arabic powder and the like can be mentioned. The amount of the binder to be used is preferably 0.1-50 parts by weight, more preferably 0.5-40 parts by weight, per 100 parts by weight of the solid preparation.

Preferable examples of the lubricant include magnesium stearate, calcium stearate, talc, sucrose esters of fatty acids, sodium stearyl fumarate and the like.

As the coloring agent, for example, food colors such as Food Yellow No. 5, Food Red No. 2, Food Blue No. 2 and the like, food lake colors, ferric oxide and the like can be mentioned.

As the pH regulator, citrate, phosphate, carbonate, tartrate, fumarate, acetate, amino acid salt and the like can be mentioned.

As the surfactant, sodium lauryl sulfate, polysorbate 80, polyoxyethylene (160) polyoxypropylene (30) glycol and the like can be mentioned.

As the release-sustaining agent, for example, cellulose polymers such as hydroxypropyl cellulose, hydroxypropylmethyl cellulose (preferably hydroxypropylmethyl cellulose 2910, hydroxypropylmethyl cellulose 2208 and the like), cellulose acetate (preferably cellulose acetate having an acetyl content of 39.3-40%), cellulose diacetate, cellulose triacetate, cellulose acetate propionate, ethyl cellulose, sodium carboxymethyl cellulose, crystalline cellulose, sodium carboxymethyl cellulose and the like; sodium alginate, carboxyvinyl polymer; acrylic acid polymers such as aminoalkylmethacrylate copolymer RS [Eudragit RS (trademark), Rohm Pharma], ethyl acrylate-methyl methacrylate copolymer suspension [Eudragit NE (trademark), Rohm Pharma] and the like; and the like can be mentioned. The release-sustaining agent may contain, for example, flux enhancers (e.g., sodium chloride, potassium chloride, sucrose, sorbitol, mannitol, polyethylene glycol (preferably polyethylene glycol 400 and the like), propylene glycol, hydroxypropyl cellulose, hydroxypropylmethyl cellulose, hydroxypropylmethyl cellulose phthalate, cellulose acetate phthalate, polyvinyl alcohol, methacrylic acid copolymer), plasticizers (e.g., triacetine, acetylated monoglyceride, grape seed oil, olive oil, sesame oil, acetyltributyl citrate, acetyltriethyl citrate, glycerin sorbitol, diethyl oxalate, diethyl maleate, diethyl fumarate, dibutyl succinate, diethyl malonate, dioctyl phthalate, dibutyl sebacate, triethyl citrate, tributyl citrate, glycerol tributyrate) and the like. Preferable examples of the release-sustaining agent include (1) a semipermeable membrane coating containing cellulose acetate (preferably cellulose acetate having an acetyl content of 39.3-40%), polyethylene glycol (preferably polyethylene glycol 400 and the like) and triacetine; (2) a release-sustaining composition containing sodium carboxymethyl cellulose, hydroxypropylmethyl cellulose 2910, hydroxypropylmethyl cellulose 2208 and microcrystalline cellulose; and the like can be mentioned.

As the stabilizer, for example, tocopherol, tetrasodium edetate, nicotinamide, cyclodextrins and the like can be mentioned.

As the sour agent, for example, ascorbic acid, citric acid, tartaric acid, malic acid and the like can be mentioned.

As the flavor, for example, menthol, peppermint oil, lemon oil, vanillin and the like can be mentioned.

As the fluidizing agent, for example, light silicic anhydride, hydrated silicon dioxide and the like can be mentioned.

The above-mentioned additives may be used in a mixture of two or more kinds thereof in an appropriate ratio.

The core of the present invention preferably contains an active ingredient. As used herein, as the active ingredient, therapeutic agents for diabetes, therapeutic agents for diabetic complications, therapeutic agents for hyperlipidemia, antihypertensive agents, antiobesity agents, diuretics, antithrombotic agents and the like can be mentioned. These active ingredients may be a low-molecular-weight compound, a high-molecular-weight protein, polypeptide or antibody, a vaccine and the like. The active ingredient may be a mixture of two or more kinds of components in an appropriate ratio.

Use of a core containing an active ingredient as the core of the present invention in this way affords superior effects such as 1) enhancing the action of pioglitazone hydrochloride or an active ingredient (synergistic effect on the action of pharmaceutical agent), 2) reducing the dose of pioglitazone hydrochloride or an active ingredient (effect of reducing the dose of pharmaceutical agent as compared to a single drug administration), 3) reducing the secondary action of pioglitazone hydrochloride or an active ingredient, and the like.

Examples of the therapeutic agents for diabetes include insulin preparations (e.g., animal insulin preparations extracted from the pancreas of cattle, swine; human insulin preparations synthesized by genetic engineering techniques using *Escherichia coli* or yeast; zinc insulin; protamine zinc insulin; fragments or derivatives of insulin (e.g., INS-1 etc.) and the like), insulin sensitizers (e.g., pioglitazone or its hydrochloride, rosiglitazone or its maleate, GI-262570, reglixane (JTT-501), netoglitazone (MCC-555), YM-440, KRP-297, CS-011, FK-614, compounds described in WO99/58510 (e.g., (E)-4-[4-(5-methyl-2-phenyl-4-oxazolylmethoxy)benzyloxyimino]-4-phenylbutyric acid), ragaglitazar (NN-622), tesaglitazar (AZ-242), BMS-298585, ONO-5816, LM-4156, BM-13-1258, MBX-102, GW-1536, LY-519818 etc.), α-glucosidase inhibitors (e.g., voglibose, acarbose, miglitol, emiglitate etc.), biguanides (e.g., phenformin, metformin, buformin, or a salt thereof (e.g., hydrochloride, fumarate, succinate) etc.), insulin secretagogues [sulfonylureas (e.g., tolbutamide, glibenclamide, gliclazide, chlorpropamide, tolazamide, acetohexamide, glyclopyramide, glimepiride, glipizide, glybuzole etc.), repaglinide, nateglinide, mitiglinide or calcium salt hydrate thereof, GLP-1 etc.], dipeptidylpeptidase IV inhibitors (e.g., NVP-DPP-278, PT-100, NVP-DDP-728, LAF237, etc.), β3 agonists (e.g., CL-316243, SR-58611-A, UL-TG-307, SB-226552, AJ-9677, BMS-196085, AZ-40140 etc.), amylin agonists (e.g., pramlintide etc.), phosphotyrosine phosphatase inhibitors (e.g., vanadic acid etc.), gluconeogenesis inhibitors (e.g., glycogen phosphorylase inhibitors, glucose-6-phosphatse inhibitors, glucagon antagonists etc.) and SGLUT (sodium-glucose cotransporter) inhibitors (e.g., T-1095 etc.).

Examples of the therapeutic agents for diabetic complications include aldose reductase inhibitors (e.g., tolrestat, epalrestat, zenarestat, zopolrestat, minalrestat, fidarestat (SNK-860), CT-112 etc.), neurotrophic factors (e.g., NGF, NT-3, BDNF etc.), neurotrophin production-secretion promoters [e.g., neurotrophin production-secretion promoters described in WO01/14372 (e.g., 4-(4-chlorophenyl)-2-(2-methyl-1-imidazolyl)-5-(3-(2-methylphenoxy)propyl)oxazole and the like)], PKC inhibitors (e.g., LY-333531 etc.), AGE inhibitors (e.g., ALT946, pimagedine, pyratoxanthine, N-phenacylthiazolium bromide (ALT766), EXO-226 etc.), active oxygen scavengers (e.g., thioctic acid etc.) and cerebral vasodilators (e.g., tiapride, mexiletine etc.).

Examples of the therapeutic agents for hyperlipidemia include HMG-CoA reductase inhibitors (e.g., pravastatin, simvastatin, lovastatin, atorvastatin, fluvastatin, lipantil, cerivastatin, itavastatin, ZD-4522, or their salts (e.g., sodium salts, calcium salts, etc.), etc.), fibrate compounds (e.g., bezafibrate, beclofibrate, binifibrate, cyprofibrate, clinofibrate, clofibrate, clofibric acid, etofibrate, fenofibrate, gemfibrozil, nicofibrate, pirifibrate, ronifibrate, simfibrate, theofibrate etc.), squalene synthase inhibitors (e.g., compounds described in WO97/10224 (e.g., N-[[(3R,5S)-1-(3-acetoxy-2,2-dimethylpropyl)-7-chloro-5-(2,3-dimethoxyphenyl)-2-oxo-1,2,3,5-tetrahydro-4,1-benzooxazepin-3-yl]acetyl]piperidine-4-acetic acid, etc.), ACAT inhibitors (e.g., Avasimibe, Eflucimibe etc.), anion exchange resins (e.g., colestyramine etc.), probucol, nicotinic acid drugs (e.g., nicomol, niceritrol etc.), ethyl icosapentate, plant sterols (e.g., soysterol, γ-oryzanol etc.) and the like.

Examples of the antihypertensive agents include angiotensin converting enzyme inhibitors (e.g., captopril, enalapril, delapril etc.), angiotensin II antagonists (e.g., candesartan cilexetil, losartan, eprosartan, valsartan, telmisartan, irbesartan, tasosartan etc.), calcium antagonists (e.g., manidipine, nifedipine, nicardipine amlodipine, efonidipine etc.), potassium channel openers (e.g., levcromakalim, L-27152, AL 0671, NIP-121 etc.), clonidine and the like.

Examples of the antiobesity agents include central antiobesity agents (e.g., dexfenfluramine, fenfluramine, phentermine, sibutramine, amfepramone, dexamphetamine, mazindol, phenylpropanolamine, clobenzorex etc.), pancreatic lipase inhibitors (e.g., orlistat etc.), β3 agonists (e.g., CL-316243, SR-58611-A, UL-TG-307, SB-226552, AJ-9677, BMS-196085, AZ-40140 etc.), peptidic anorexiants (e.g., leptin, CNTF (Ciliary Neurotropic Factor) etc.), cholecystokinin agonists (e.g., lintitript, FPL-15849 etc.) and the like.

Examples of the diuretics include xanthine derivatives (e.g., sodium salicylate and theobromine, calcium salicylate and theobromine etc.), thiazide preparations (e.g., ethiazide, cyclopenthiazide, trichloromethyazide, hydrochlorothiazide, hydroflumethiazide, benzylhydrochlorothiazide, penflutizide, polythiazide, methyclothiazide etc.), antialdosterone preparations (e.g., spironolactone, triamterene etc.), carbonate dehydratase inhibitors (e.g., acetazolamide etc.), chlorobenzenesulfonamide preparations (e.g., chlortalidone, mefruside, indapamide etc.), azosemide, isosorbide, etacrynic acid, piretanide, bumetanide, furosemide and the like.

Examples of the antithrombotic agents include heparin (e.g., heparin sodium, heparin calcium, dalteparin sodium etc.), warfarin (e.g., warfarin potassium etc.), anti-thrombin drugs (e.g., aragatroban etc.), thrombolytic agents (e.g., urokinase, tisokinase, alteplase, nateplase, monteplase, pamiteplase etc.), platelet aggregation inhibitors (e.g., ticlopidine hydrochloride, cilostazol, ethyl icosapentate, beraprost sodium, sarpogrelate hydrochloride etc.) and the like.

The active ingredient is preferably a therapeutic agent for diabetes, more preferably a biguanide, particularly preferably metformin or a salt thereof (preferably metformin hydrochloride).

The amount of the active ingredient in the core of the present invention is, for example, 0.1-100 parts by weight, preferably 1-99 parts by weight, per 100 parts by weight of the core of the present invention.

The core of the present invention is preferably a tablet containing an active ingredient (preferably a therapeutic agent for diabetes, more preferably a biguanide, particularly preferably metformin hydrochloride). The shape of the tablet may be any from pill, caplet, oblong and the like. The tablet can be produced by, for example, mixing or granulating the active ingredient with the aforementioned additives, and then compression-molding the obtained mixture or granules after mixing, according to methods conventionally employed in the field of pharmaceutical preparation.

Here, mixing is done using, for example, a mixer such as a V-type mixer, a tumbler mixer and the like, and granulation is done using, for example, a high speed mixer granulator, a fluid bed granulator-dryer and the like. For compression-molding, punching is done generally at a pressure of 5-35 kN/cm² using a single punch tableting machine, rotary tableting machine and the like.

When the active ingredient contained in the core of the present invention is not a pharmaceutical agent for a single administration per day (e.g., in the case of a pharmaceutical agent for administration twice or three times a day), the core containing said active ingredient is preferably a sustained release preparation.

When the mixing stability of pioglitazone hydrochloride and active ingredient contained in the core of the present invention is poor, the core containing the active ingredient may be coated with the aforementioned coating base and the like.

The core of the present invention is more preferably a sustained release preparation (preferably tablet) containing a biguanide (preferably metformin hydrochloride). As such preparation, for example, a controlled release pharmaceutical agent tablet described in WO99/47125, a two-layer controlled release delivery system described in WO99/47128, a controlled release oral pharmaceutical agent described in USP6340475 and the like can be mentioned.

As a sustained release preparation containing a biguanide,
(1) a biguanide-containing tablet coated with a semipermeable membrane coating, which contains cellulose acetate (preferably cellulose acetate having an acetyl content of 39.3-40%), polyethylene glycol (preferably polyethylene glycol 400 and the like) and triacetine (said semipermeable membrane coating may have a hole or pore);
(2) a tablet obtained by mixing a release-sustaining composition containing sodium carboxymethyl cellulose, hydroxypropylmethyl cellulose 2910, hydroxypropylmethyl cellulose 2208 and crystalline cellulose with a biguanide, and then compression-molding the mixture, and the like are preferable.

In the production method of the present invention, the coating is done according to known methods. For example, coating is done using a film coating equipment.

In addition, coating is done such that the core of the present invention is generally 50-99 parts by weight, preferably 70-99 parts by weight, more preferably 70-98 parts by weight, per 100 parts by weight of the obtained coated preparation.

Furthermore, the "preparation coated with pioglitazone hydrochloride" obtained according to the production method of the present invention (hereinafter sometimes to be abbreviated as a coated preparation of the present invention) may be coated with the aim of improving preparation strength, coloring and the like of the coated preparation. Such coating can be applied according to a known method and using, for example, the aforementioned coating base and the like.

As the dosage form of the coated preparation of the present invention, for example, tablet, capsule, granule, powder, troche and the like can be mentioned. The dosage form of the coated preparation is preferably a tablet. The shape of the tablet may be any from pill, caplet, oblong and the like. In addition, a mark or a letter may be printed on the tablet for identifiability, and a separating line may be made to facilitate division.

The amount of the active ingredient in the coated preparation of the present invention is, for example, generally 0.01-99 parts by weight, preferably 0.1-99 parts by weight, per 100 parts by weight of the coated preparation. Particularly, when the active ingredient is a biguanide (preferably metformin hydrochloride), the amount of the biguanide in the coated preparation is, for example, generally 5-98 parts by weight, preferably 15-96 parts by weight, per 100 parts by weight of the coated preparation.

The amount of pioglitazone hydrochloride in the coated preparation of the present invention is, for example, generally 0.01-25 parts by weight, preferably 0.01-15 parts by weight, more preferably 0.5-15 parts by weight, per 100 parts by weight of the coated preparation.

The coated preparation of the present invention can be administered orally and safely to mammals (e.g., mouse, rat, rabbit, cat, dog, bovine, horse, monkey, human and the like).

The coated preparation of the present invention is superior in the characteristics of the preparation, such as dissolution property of pioglitazone hydrochloride and the like, and is useful as a prophylactic or therapeutic agent for, for example, diabetes (e.g., type-1 diabetes, type-2 diabetes, gestational diabetes etc.), hyperlipidemia (e.g., hypertriglyceridemia, hypercholesterolemia, hypo-HDL-emia, postprandial hyperlipidemia etc.), impaired glucose tolerance [IGT (Impaired Glucose Tolerance)], diabetic complications [e.g., neuropathy, nephropathy, retinopathy, cataract, macroangiopathy, osteopenia, hyperosmolar diabetic coma, infectious disease (e.g., respiratory infection, urinary tract infection, gastrointestinal infection, dermal soft tissue infections, inferior limb infection etc.), diabetic gangrene, xerostomia, hypacusis, cerebrovascular disorder, peripheral blood circulation disorder etc.], obesity, osteoporosis, cachexia (e.g., cancerous cachexia, tuberculous cachexia, diabetic cachexia, blood disease cachexia, endocrine disease cachexia, infectious disease cachexia or cachexia due to acquired immunodeficiency syndrome), fatty liver, hypertension, polycystic ovary syndrome, kidney disease (e.g., diabetic nephropathy, glomerular nephritis, glomerulosclerosis, nephrotic syndrome, hypertensive nephrosclerosis, end stage kidney disease etc.), muscular dystrophy, myocardial infarction, angina pectoris, cerebrovascular accident (e.g., cerebral infarction, cerebral apoplexy), insulin resistance syndrome, Syndrome X, hyperinsulinemia, hyperinsulinemia-induced sensory disorder, tumor (e.g., leukemia, breast cancer, prostate cancer, skin cancer etc.), irritable bowel syndrome, acute or chronic diarrhea, inflammatory diseases [e.g., Alzheimer's disease, chronic rheumatoid arthritis, spondylitis deformans, osteoarthritis cleformans, lumbagor pain, gout, postoperative or traumatic inflammation, remission of tumentia, neuralgia, pharyngolaryngitis, cystitis, hepatitis (inclusive of nonalcoholic steatohepatitis), pneumonia, pancreatitis, inflammatory bowel disease, ulcerative colitis, etc.], visceral obesity syndrome, arteriosclerosis (e.g., atherosclerosis etc.) and the like.

The coated preparation of the present invention is also useful for the secondary prevention of the above-mentioned various diseases (e.g., secondary prevention of cardiovascular event such as cardiac infarction etc.) and suppression of progression (e.g., suppression of progression of impaired glucose tolerance into diabetes, suppression of progression of arteriosclerosis in diabetic patients).

The dose of the coated preparation of the present invention is 7.5-60 mg/day, preferably 15-60 mg/day, more preferably 15-45 mg/day, based on the amount of pioglitazone hydrochloride, for an adult (body weight 60 kg).

When the coated preparation of the present invention is obtained using a core containing an active ingredient, the coated preparation preferably contains an effective amount of the active ingredient. For example, the effective amount when the active ingredient is a biguanide (preferably metformin hydrochloride) is 125-2550 mg/day, preferably 250-2550 mg/day, for an adult (body weight 60 kg).

The coated preparation of the present invention may be used in combination with one or more pharmaceutical agents selected from therapeutic agents for diabetes, therapeutic agents for diabetic complications, therapeutic agents for hyperlipidemia, antihypertensive agents, antiobesity agents, diuretics, antithrombotic agents and the like (hereinafter sometimes to be abbreviated as a concomitant drug). As such concomitant drugs, those exemplified above as the active ingredient can be used. The time of administration of the coated preparation of the present invention and that of the concomitant drug are not limited, and they may be administered simultaneously or at staggered times to the administration subject. The dose of the concomitant drug can be appropriately determined based on the dose clinically employed. In addition, the mixing ratio of the coated preparation of the present invention and the concomitant drug can be appropriately determined according to the administration subject, administration route, target disease, condition, combination, and the like. For example, when the administration subject is a human, the concomitant drug may be used in an amount of 0.01 to 100 parts by weight per 1 part by weight of the coated preparation.

Use of the concomitant drug in this way provides superior effects such as 1) enhancing the action of the coated preparation of the present invention or the concomitant drug (synergistic effect on the action of the pharmaceutical agents), 2) reducing the dose of the coated preparation of the present invention or the concomitant drug (effect of reducing the dose of pharmaceutical agents as compared to a single drug administration), 3) reducing the secondary action of the coated preparation of the present invention or the concomitant drug, and the like.

The present invention further relates to "a method for improving dissolution of pioglitazone hydrochloride from a preparation coated with pioglitazone hydrochloride, which comprises, when producing said preparation, coating a dispersion of pioglitazone hydrochloride in an organic solvent, which contains a coating base soluble in organic solvents"

Use of the production method of the present invention when producing a preparation coated with pioglitazone hydrochloride can afford a coated preparation superior in the dissolution property of pioglitazone hydrochloride.

The present invention moreover relates to "a coated preparation obtained according to the production method of the present invention, which shows elution of not less than 50% of pioglitazone hydrochloride in 15 minutes in a dissolution test by a rotating basket method using a hydrochloric acid-potassium chloride buffer (pH 2.0) as a test solution at 37°C, 100 rpm". As used herein, the dissolution test is performed according to the method described in The Japanese Pharmacopoeia 14th Edition. The "hydrochloric acid-potassium chloride buffer (pH 2.0)" used as a test solution can be prepared according to a known method. The amount of the hydrochloric acid-potassium chloride buffer used as a test solution is generally 900 mL.

The "coated preparation obtained according to the production method of the present invention, which shows elution of not less than 50% of pioglitazone hydrochloride in 15 minutes in a dissolution test by a rotating basket method using a hydrochloric acid-potassium chloride buffer (pH 2.0) as a test solution at 37°C, 100 rpm" can be administered orally and safely to mammals (e.g., mouse, rat, rabbit, cat, dog, bovine, horse, monkey, human and the like), as the aforementioned coated preparation of the present invention, wherein the target disease, dose and the like are the same as those in the aforementioned coated preparation of the present invention.

The present invention moreover relates to "a coated preparation obtained according to the production method of the present invention, which shows elution of not less than 50% of pioglitazone hydrochloride in 15 minutes in a dissolution test by a puddle method using a hydrochloric acid-potassium chloride buffer (pH 2.0) as a test solution at 37°C, 50 rpm". As used herein, the dissolution test is performed according to the method described in The Japanese Pharmacopoeia 14th Edition. The "hydrochloric acid-potassium chloride buffer (pH 2.0)" used as a test solution can be prepared according to a known method. The amount of the hydrochloric acid-potassium chloride buffer used as a test solution is generally 900 mL.

The "coated preparation obtained according to the production method of the present invention, which shows elution of not less than 50% of pioglitazone hydrochloride in 15 minutes in a dissolution test by a puddle method using a hydrochloric acid-potassium chloride buffer (pH 2.0) as a test solution at 37°C, 50 rpm" can be administered orally and safely to mammals (e.g., mouse, rat, rabbit, cat, dog, bovine, horse, monkey, human and the like), as the aforementioned coated preparation of the present invention, wherein the target disease, dose and the like are the same as those in the aforementioned coated preparation of the present invention.

The present invention is explained in detail in the following by referring to Examples, Reference Example and Experimental Examples, which are not to be construed as limitative.

As the preparation additives (e.g., D-mannitol, corn starch, hydroxypropyl cellulose, magnesium stearate, crystalline cellulose, polyvinylpyrrolidone polyethylene glycol 6000, titanium oxide) used in the following Examples and Reference Example, those capable of meeting the standards of The Japanese Pharmacopoeia 14th Edition were used.

### Examples

### Example 1

Polyvinylpyrrolidone (K-30) (18.9 g), polyethylene glycol 6000 (3.6 g) and pioglitazone hydrochloride (7.5 g, average particle size 12 µm) were dispersed in ethanol (170 g) to give a coating solution.

Glucophage XR tablets (trademark) (300 g) (sustained-release tablet containing 500 mg of metformin hydrochloride)(manufactured by Bristol-Myers Squibb) were fed in a film coating equipment (Hicoater-Mini, Freund Industrial Co. Ltd.) and coated with the aforementioned coating solution (spray speed: 1.0 g/min) at an entrance temperature of 80°C to give a coated preparation containing 500 mg of metformin hydrochloride/16.53 mg of pioglitazone hydrochloride.

### Example 2

Hydroxypropyl cellulose (26.4 g, Grade L, Nippon Soda Co., Ltd.), polyethylene glycol 6000 (1.32 g), titanium oxide (2.64 g) and pioglitazone hydrochloride (16.5 g) were dispersed in ethanol (297 g) to give a coating solution.

The tablets (300 g) obtained in Reference Example 1 were fed in a film coating equipment (Hicoater-Mini, Freund Industrial Co. Ltd.) and coated with the aforementioned coating solution at an entrance temperature of 60°C to give a coated preparation weighing 260 mg per tablet.

### Example 3

Hydroxypropyl cellulose (26.4 g, Grade SSL, Nippon Soda Co., Ltd.), polyethylene glycol 6000 (1.32 g), titanium oxide (2.64 g) and pioglitazone hydrochloride (16.5 g) were dispersed in ethanol (297 g) to give a coating solution.

The tablets (250 g) obtained in Reference Example 1 were fed in a film coating equipment (Hicoater-Mini, Freund Industrial Co. Ltd.) and coated with the aforementioned coating solution at an entrance temperature of 60°C to give a coated preparation weighing 259.7 mg per tablet.

### Reference Example 1

D-mannitol (2176 g) and corn starch (918 g) were charged in a fluid bed granulator-dryer (FD-3S, manufactured by POWREX CORPORATION) and granulated while spraying an aqueous solution (1700 g) containing hydroxypropyl cellulose (102 g), which was followed by a drying step to give granules. Crystalline cellulose (160.2 g) and magnesium stearate (32 g) were added to the obtained granule powder (3012 g) and mixed. The obtained granule powder mixture was tableted by a tableting machine (Correct 19K, manufactured by Kikusui Seisakusho Ltd.)(tablet size: 8.5 mm φ, compression pressure 9KN/punch) to give tablets weighing 244 mg per tablet.

### Experimental Example 1

The coated preparation obtained in the aforementioned Example 1 was evaluated for the dissolution property of pioglitazone hydrochloride by a rotating basket method (100 rpm) using a 0.3 M hydrochloric acid-potassium chloride buffer (900 mL, 37°C, pH 2.0). The results are shown in Table 1.

**[Table 1]**

| Dissolution (%) of pioglitazone hydrochloride | | | | |
|---|---|---|---|---|
| Time | 15 min. | 30 min. | 45 min. | 60 min. |
| Example 1 | 69.3 | 77.7 | 85.5 | 91.4 |

As shown in Table 1, the coated preparation obtained by the production method of the present invention showed superior dissolution property of pioglitazone hydrochloride.

### Experimental Example 2

The coated preparations obtained in the aforementioned Example 2 and Example 3 were evaluated for the dissolution property of pioglitazone hydrochloride by a puddle method (50 rpm) using a 0.3 M hydrochloric acid-potassium chloride buffer (900 mL, 37°C, pH 2.0). The results are shown in Table 2.

**[Table 2]**

| Dissolution (%) of pioglitazone hydrochloride | | | | |
|---|---|---|---|---|
| Time | 15 min. | 30 min. | 45 min. | 60 min. |
| Example 2 | 69 | 93 | 99 | 101 |
| Example 3 | 72 | 97 | 99 | 100 |

As shown in Table 2, the coated preparations obtained by the production method of the present invention showed superior dissolution property of pioglitazone hydrochloride.

### INDUSTRIAL APPLICABILITY

The coated preparation obtained by the production method of the present invention is useful as a therapeutic agent for diabetes and the like and superior in the characteristics of the preparation such as dissolution property of pioglitazone hydrochloride and the like.

## Claims

1. A production method of a coated preparation, which comprises coating with a dispersion of pioglitazone hydrochloride in an organic solvent, which contains a coating base soluble in organic solvents.

2. A coated preparation obtained according to the production method of claim 1.

3. The production method of claim 1, which comprises coating a core containing an active ingredient with a dispersion of pioglitazone hydrochloride in an organic solvent, which contains a coating base soluble in organic solvents.

4. The production method of claim 3, wherein the active ingredient is a therapeutic agent for diabetes.

5. The production method of claim 4, wherein the therapeutic agent for diabetes is a biguanide.

6. The production method of claim 5, wherein the biguanide is metformin hydrochloride.

7. The production method of claim 1, wherein the organic solvent is an alcohol or a ketone.

8. The production method of claim 1, wherein the organic solvent is ethanol.

9. The production method of claim 1, wherein the coating base soluble in organic solvents is polyvinylpyrrolidone.

10. A method for improving dissolution of pioglitazone hydrochloride from a preparation coated with pioglitazone hydrochloride, which comprises, when producing said preparation, coating with a dispersion of pioglitazone hydrochloride in an organic solvent, which contains a coating base soluble in organic solvents.

11. A coated preparation obtained according to the production method of claim 1, which shows elution of not less than 50% of pioglitazone hydrochloride in 15 minutes in a dissolution test by a rotating basket method using a hydrochloric acid-potassium chloride buffer (pH 2.0) as a test solution at 37°C, 100 rpm.

12. A coated preparation obtained according to the production method of claim 1, which shows elution of not less than 50% of pioglitazone hydrochloride in 15 minutes in a dissolution test by a puddle method using a hydrochloric acid-potassium chloride buffer (pH 2.0) as a test solution at 37°C, 50 rpm.
